(19) Europäisches Patentamt
European Patent Office
Office européen des brevets

(11) Numéro de publication: **0 402 203 A2**

(12) **DEMANDE DE BREVET EUROPEEN**

(21) Numéro de dépôt: **90401452.9**

(51) Int. Cl.$^5$: **A61K 37/64, A61K 37/02**

(22) Date de dépôt: **31.05.90**

(30) Priorité: **05.06.89 FR 8907410**

(43) Date de publication de la demande: **12.12.90 Bulletin 90/50**

(84) Etats contractants désignés: **AT BE CH DE DK FR GB IT LI LU NL SE**

(71) Demandeur: **SANOFI S.A.**
**40, Avenue George V**
**F-75008 Paris(FR)**

(72) Inventeur: **Nisato, Dino**
**2, rue de Terre Rouge**
**F-34680 St Georges d'Orques(FR)**
Inventeur: **Le Fur, Gérard**
**19 ter, rue des Carrières**
**F-95160 Montmorency(FR)**

(74) Mandataire: **Gillard, Marie-Louise et al**
**Cabinet Beau de Loménie 55, Rue d'Amsterdam**
**F-75008 Paris(FR)**

(54) **Utilisation d'un dérivé de la statine dans le traitement des affections oculaires.**

(57) La présente invention a pour objet l'utilisation du N-(pyridyl-3 propionyl)phénylalanyl-histidyl-(cyclohéxyl)-statyl-N (dihydroxy-1,3 méthyl-2 propyl)isoleucinamide qui répond à la formule :

$$\text{N-(Pyridyl-3)-3 propionyl-Phe-His-(cyclohéxyl)Sta-Ile-NH-}\underset{\displaystyle CH_2OH}{\overset{\displaystyle CH_2OH}{\overset{|}{\underset{|}{C}}}}\text{-}CH_3 \qquad (I)$$

dans laquelle : Phe représente le résidu de la (L) phénylalanine ; His représente le résidu de la (L) histidine ; Ile représente le résidu de la (L) isoleucine et, (cyclohéxyl)Sta représente le résidu de la cyclohéxyle statine, c'est-à-dire de l'acide (3S, 4S) amino-4 cyclohéxyl-5 hydroxy-3 pentanoïque, pour la préparation de médicaments utiles dans le traitement des affections oculaires.
Application : traitement des affections oculaires.

EP 0 402 203 A2

## Utilisation d'un dérivé de la statine dans le traitement des affections oculaires.

La présente invention a pour objet l'utilisation d'un dérivé de la statine dans le traitement des affections oculaires telles que le glaucome et la rétinopathie diabétique.

Le glaucome est une affection oculaire caractérisée, entre autres symptomes, par une augmentation lente ou rapide de la pression intraoculaire. Le glaucome conduit à la destruction des fibres du nerf optique et peut entraîner la perte de la vision.

Un des traitements majeurs du glaucome consiste à réduire la pression intraoculaire. Les médicaments connus actuellement pour le traitement du glaucome présentent des difficultés d'emploi. Ainsi par exemple, la pilocarpine présente des effets secondaires locaux, tandis que des principes actifs comme l'épinéphrine ou un béta-bloquant adrénergique : le timolol, sont difficilement utilisables pour certains patients atteints de maladies cardiovasculaires ou ne supportant pas les effets cardiovasculaires généraux de ces drogues.

La demande de brevet WO 87/02581 décrit l'utilisation de certains inhibiteurs de rénine pour la préparation de médicaments destinés à réduire et à contrôler une pression intraoculaire excessive.

La rétinopathie diabétique est une autre affection oculaire qui s'observe généralement après une quinzaine d'années d'évolution du diabète. Elle apparaît au fond de l'oeil sous forme de microanévrismes et peut aboutir à une prolifération vasculaire réduisant l'acuité visuelle.

Actuellement, il existe peu de traitements pour lutter contre la rétinopathie diabétique. On peut citer par exemple l'utilisation de l'héparine associée à une fraction enzymatique de venin.

Chez certains patients atteints de diabète mellitus, on a pu constater une augmentation du taux de prorénine plasmatique (J. A. Luestscher et al. New England J. Med., 1985, 312 (22), 1412-1417).

La présente invention est relative à l'utilisation d'un inhibiteur de rénine particulièrement actif pour la préparation de médicaments destinés au traitement des affections oculaires, notamment au contrôle de la pression intraoculaire et au traitement de l'hypertension oculaire, du glaucome et de la rétinopathie diabétique.

Le composé inhibiteur de rénine particulièrement actif dont l'utilisation est l'objet de la présente invention est décrit dans la demande de brevet EP 211 744. Il s'agit du N-(pyridyl-3 propionyl)phénylalanyl-histidyl-(cyclohéxyl)statyl-N (dihydroxy-1,3 méthyl-2 propyl)isoleucinamide qui répond à la formule suivante :

$$\text{N-(Pyridyl-3)-3 propionyl-Phe-His-(cyclohéxyl)Sta-Ile-NH-}\underset{\displaystyle CH_2OH}{\overset{\displaystyle CH_2OH}{\overset{|}{\underset{|}{C}}}}\text{-}CH_3 \quad (I)$$

dans laquelle :
Phe représente le résidu de la (L) phénylalanine ;
His représente le résidu de la (L) histidine ;
Ile représente le résidu de la (L) isoleucine et
(cyclohéxyl)Sta représente le résidu de la cyclohéxyle statine, c'est-à-dire de l'acide (3S, 4S) amino-4 cyclohéxyl-5 hydroxy-3 pentanoïque.

Pour ce composé (I) on a mesuré l'inhibition de l'activité rénine plasmatique humaine à pH 7,4 selon la méthode décrite dans la demande de brevet EP 211 744. La concentration inhibitrice 50 (CI 50) du composé (I) est $10^{-12}$M.

Aucun des composés décrits dans la demande de brevet WO 87/02581 ne présente une activité aussi forte comme inhibiteur de rénine.

Pour le traitement du glaucome, l'invention se rapporte également à l'utilisation du composé I en combinaison avec un autre principe actif. Le principe actif utilisé en combinaison avec le composé I peut être soit, un agent bêta-bloquant adrénergique lui même utile pour abaisser la pression intraoculaire, soit un antiinflammatoire, notamment un antiinflammatoire stéroïdien ou un corticostéroïde, dont un effet secondaire est l'augmentation de la pression intraoculaire.

La présente invention concerne également une composition pharmaceutique contenant le composé I et destinée au traitement de l'augmentation de la pression intra-oculaire consécutive à un traitement par des antiinflammatoires stéroïdiens.

Les compositions pharmaceutiques selon la présente invention sont administrées, soit sous forme de compositions pharmaceutiques ophtalmiques adaptées à une administration topique sur l'oeil, comme des

solutions, des suspensions ou des pommades, soit sous forme de compositions pharmaceutiques adaptées à une administration systémique par voie orale, injectable, transdermique ou par inhalation.

L'invention se rapporte également à un ensemble comprenant d'une part le composé I dans un excipient ophtalmique topique et d'autre part un antiinflammatoire dans un excipient ophtalmique topique pour le traitement du glaucome.

Les formulations selon l'invention peuvent contenir de 0,000001 à 1% en poids du composé I, plus particulièrement de 0,00001 à 0,1%. Chaque unité de dosage comprend une quantité du composé I comprise entre 1 ng et 50 mg, préférentiellement entre 5 ng et 25 mg.

L'expression "contrôle de la pression intraoculaire élevée", telle qu'utilisée dans la présente description, signifie la régulation, la diminution et la modulation de la tension intraoculaire élevée qui est le premier symptôme permettant le diagnostic du glaucome. L'expression signifie également que la diminution de la pression intraoculaire obtenue selon l'invention par utilisation d'un composé I est maintenue pour une période de temps suffisante, par exemple entre l'administration de 2 doses consécutives.

Dans le cas du traitement du glaucome, le composé I peut être employé dans des compositions pharmaceutiques, soit comme seul ingrédient actif destiné à réduire la pression intraoculaire, soit en combinaison avec d'autres principes actifs également destinés à abaisser la pression intraoculaire et agissant par un mécanisme distinct, tel qu'un agent béta-bloquant adrénergique, le timolol, soùs forme de maléate, par exemple. L'utilité d'un béta-bloquant pour diminuer la pression intraoculaire est connue. Ainsi le timolol est prescrit dans le traitement topique du glaucome à raison d'une à deux gouttes par jour d'une solution à 0,25 mg/100 ml ou 0,50 mg/100 ml. On sait cependant que ce produit doit être utilisé avec précaution en raison de son activité sur le système cardiovasculaire et de ses effets secondaires.

Selon un aspect de la présente invention, il est clair que l'utilisation d'un composé I associée à celle d'un béta-bloquant, tel que le timolol maléate, peut permettre de réduire la dose utile de béta-bloquant tout en observant une réduction équivalente de la pression intraoculaire. On pourra ainsi minimiser les effets indésirables dûs au béta-bloquant.

Pour réaliser un tel traitement en association, le béta-bloquant et le composé I sont préférentiellement administrés ensemble sous forme d'une composition ophtalmique dans une formulation pharmaceutique. La forme unitaire de dosage contient préférentiellement :

- de 5 µg à 125 µg de béta-bloquant adrénergique
- et de 5 ng à 0,1 mg de composé I.

La quantité de chacun des principes actifs peut varier selon la gravité de la maladie et la réponse individuelle des patients.

Les concentrations de chacun des principes actifs de la composition permettant de réduire la pression intraoculaire sont variables, elles présentent une limite inférieure en dessous de laquelle la composition est inactive. Cette limite inférieure est environ 5 % de la dose efficace et dépend de l'age et de la taille du patient, de la gravité de la maladie, ainsi que de la puissance du béta-bloquant utilisé.

L'élévation de la pression intraoculaire, associée à une utilisation ophtalmique ou systémique des antiinflammatoires stéroïdiens, peut être réduite par l'administration du composé I. Les antiinflammatoires stéroïdiens comprennent l'hydrocortisone, la cortisone, le flunisolide, le béclométhasone, l'alclométhasone, le chlorocortolone, le diflorasone, l'alcinolide, le fluocinonide, le fluocinolone, le désoximéthasone, le médrysone, le paraméthasone, le dichloro-9,21 / (furanylcarbonyl-2) oxy-17 / hydroxy-11 méthyl-16αprégnad iène-1,4 dione-3,20 et le fluorométholone, ainsi que leurs sels et esters pharmaceutiquement acceptables.

L'élévation de la pression intraoculaire peut se produire consécutivement à tout mode d'administration de ces médicaments : une administration systémique, généralement orale, une injection locale, par exemple l'injection d'une forme retard et particulièrement lors d'une injection ophtalmique topique ou intravitreuse. Le composé I peut être administré après le traitement stéroïdien pour abaisser la pression intraoculaire élevée ou il peut être coadministré avec le stéroïde pour supprimer l'effet d'augmentation de la pression intraoculaire du stéroïde, sans cependant interférer avec son activité antiinflammatoire.

Selon la présente invention, toute combinaison des formes de dosage peut être utilisée pour administrer la combinaison du stéroïde antiinflammatoire et du composé I : les deux médicaments sous forme orale, ou les deux sous forme topique, ou l'un sous forme orale, l'autre sous forme topique, ou le stéroïde sous forme d'une injection locale et le composé I sous forme topique ; une combinaison préférée est une composition ophtalmique topique comprenant à la fois le stéroïde et le composé I.

La méthode pour réduire et contrôler la pression intraoculaire élevée, associée à l'utilisation d'un antiinflammatoire stéroïdien par voie systémique ou ophtalmique, comprend également l'administration séparée de cet agent et du composé I. C'est pourquoi la présente invention se rapporte à une trousse comprenant 2 unités séparées : une composition pharmaceutique comprenant le composé I et une composition pharmaceutique comprenant un stéroïde. Préférentiellement une telle trousse comprend une

composition ophtalmique topique du composé I et une composition pharmaceutique de stéroïde. De façon tout à fait préférentielle, la trousse comprend deux compositions ophtalmiques topiques, l'une comprenant le composé I, l'autre le stéroïde. Un avantage particulier de cette présentation est de fournir une composition à base de composé I administrable une à deux fois par jour et une composition à base de stéroïde qui peut être administrée plus souvent : toutes les heures par exemple.

Selon l'invention, la formulation topique peut contenir différentes quantités de principe actif, soit dans la même composition, soit administrées séparément.

Le composé I représente de 0,000001 % à environ 1 % en poids du médicament, plus particulièrement de 0,00001 à 0,1 %. Une forme unitaire de dosage comprend de 1 ng à 50 mg, préférentiellement entre 5 ng et 25 mg. Dans chaque cas individuel, la quantité à administrer et la fréquence d'administration dépendent de la puissance du stéroïde choisi, de la gravité de l'affection oculaire à traiter et de la réponse du patient.

Le stéroïde représente de 0,05 % à environ 1,5 % en poids du médicament. Une forme unitaire de dosage comprend de 20 ug à 600 ug à appliquer sur l'oeil. Dans chaque cas individuel, la quantité à administrer et la fréquence d'administration dépendent de la puissance du stéroïde choisi, de la gravité de la maladie et de la réponse du patient.

Selon un autre aspect de l'invention, les deux principes actifs, à savoir le composé I et le stéroïde, sont administrés simultanément et contenus dans la même forme pharmaceutique, chacun étant présent dans la forme pharmaceutique à sa concentration préférée. Lorsque le stéroïde est administré par voie systémique ou topique, autre que ophtalmique, son dosage peut varier en fonction des critères précédemment décrits et connus par l'homme de l'art. Pour les solutions et les suspensions, il faut également tenir compte du volume représenté par une goutte de composition pharmaceutique.

L'effet d'abaissement de la pression intraoculaire d'une composition pharmaceutique selon l'invention peut être déterminé chez l'animal, par exemple chez le lapin, dans un test qui prévoit l'administration orale de quantités importantes d'eau, tel que décrit par exemple dans Arch. Ophthal., 1969, 82, 381-384 ou dans J. Ocul. Pharmacol., 1985, 1 (2), 161-168.

Ainsi, le composé (I) décrit dans la présente demande a été étudié pour son effet sur la pression intraoculaire chez le lapin. L'administration de ce composé sous forme de collyre, permet un rétablissement rapide de la pression intraoculaire à sa valeur normale, après que ladite pression ait été augmentée par injection intraveineuse d'une solution de glucose à 5 %.

Pour préparer des formulations convenables, les compositions pharmaceutiques peuvent être mélangées avec un véhicule convenable, soit pour une administration ophtalmique topique, soit pour une administration par voie générale. Comme véhicules pharmaceutiques acceptables pour une administration ophtalmique, on peut citer l'eau, un mélange d'eau et de solvants miscibles à l'eau comme les alcanols inférieurs, les huiles végétales ou les huiles minérales, comprenant de 0,5 à 5 % en poids d'hydroxyéthylcellulose, d'oléate d'éthyle, de carboxyméthylcellulose, de polyvinylpyrrolidone et d'autres polymères solubles dans l'eau, non toxiques et compatibles avec une utilisation ophtalmique, par exemple des dérivés de la cellulose, tels que la méthylcellulose, un dérivé d'un métal alcalin de carboxyméthylcellulose, l'hydroxyméthylcellulose,l'hydroxyéthylcellulose, l'hydroxypropylméthylcellulose, des acrylates tels que des sels d'acides polyacryliques, des éthylacrylates, des polyacrylamides, des produits naturels tels que la gélatine, les alginates, les pectines, le tragacanthe, le karaya, le xanthane, le carraghenane, l'agar , l'acacia, des dérivés d'amidon comme l'acétate d'amidon, les éthers d'hydroxyéthylamidon, l'hydroxypropylamidon, ainsi que d'autres dérivés synthétiques comme le polyvinylalcool, polyvinylpyrrolidone, polyvinylméthyléther, polyéthylèneoxyde, le carbopol neutre ou des mélanges de ces polymères. La préparation pharmaceutique peut aussi contenir des substances auxiliaires non toxiques comme des émulsifiants, des conservateurs, des agents de mouillage, des agents texturants et d'autres comme par exemple les polyéthylènes glycols 200, 300, 400, 600, les carbowax 1000, 1500, 4000, 6000, 10000 des produits antibactériens comme des ammonium quaternaires, des sels phénylmercuriques connus pour avoir des propriétés stérilisantes à froid sans être agressifs, le timérosal, le propylparaben, l'alcool benzylique, le phényl éthanol, des agents isotoniques comme un chlorure de métal alcalin, des tampons borate, acétate ou gluconate, des antioxydants comme le métabisulfite de sodium, l'hydroxyanisole butylé, l'hydroxytoluène butylé ou des agents semblables et d'autres agents conventionnellement utilisés comme le monolaurate de sorbitan, l'oléate de triéthanolamine, le monopalmitate de polyéthylène sorbitan, un sel de métal alcalin du dioctyl sulfosuccinate, le monothioglycérol, l'acide éthylènediamine tétracétique ou autre.

De plus, des excipients ophtalmiques acceptables peuvent être utilisés comme le tampon phosphate, l'acide borique isotonique, un chlorure alcalin isotonique, ou le tris par exemple.

La préparation pharmaceutique peut également être une suspension dans laquelle les particules sont des polymères solubles dans l'eau ou insolubles dans l'eau. Une telle suspension peut contenir des

microformes telles que des microparticules ou des nanoparticules.

Les compositions selon l' invention peuvent contenir des agents thérapeutiques additionnels en plus du composé I. Ainsi des antibiotiques, des anesthésiques ou d'autres agents peuvent être présents.

Les exemples suivants illustrent l'invention sans toutefois la limiter.

| Exemple 1 | |
|---|---|
| Solution topique Composé I | 1 mg |
| chlorure de sodium | 9 mg |
| eau distillée q.s.p. | 1 ml |
| NaOH 1 N q.s. | pH = 5,5 |

Les constituants de la solution sont mélangés dans les conditions habituelles pour obtenir une solution ophtalmique.

| Exemple 2 | |
|---|---|
| Solution topique Composé I | 1 mg |
| timolol maléate | 5 mg |
| chlorure de benzalkonium | 0,1 mg |
| chlorure de sodium | 9 mg |
| eau distillée q.s.p. | 1 ml |
| Na OH, 1 N q.s. | pH = 5,5 |

Les constituants de la solution sont mélangés dans les conditions habituelles pour obtenir une solution ophtalmique.

## Revendications

1. Utilisation du N-(pyridyl-3 propionyl)phénylalanyl-histidyl-(cyclohéxyl)statyl-N (dihydroxy-1,3 méthyl-2 propyl)isoleucinamide pour la préparation d'un médicament destiné au traitement des affections oculaires.

2. Utilisation du composé selon la revendication 1, caractérisé en ce qu'il est utilisé en combinaison avec des excipients ophtalmiques acceptables pour un usage topique.

3. utilisation du composé selon la revendication 1, caractérisé en ce qu'il est utilisé en combinaison avec des excipients acceptables pour une administration systémique.

4. utilisation du composé selon l'une des revendications 1, 2 ou 3 en combinaison avec un agent béta-bloquant adrénergique.

5. Utilisation selon la revendication 4 du composé selon la revendication 1, caractérisé en ce que le béta-bloquant adrénergique est le timolol maléate.

6. Utilisation du composé selon l'une des revendications 1 à 5 en combinaison avec un antiinflammatoire stéroïdien.

7. Une trousse comprenant dans des parties séparées, d'une part le composé selon la revendication 1 sous une forme ophtalmique topique, d'autre part un stéroïde sous une forme pharmaceutiquement acceptable.

8. Une trousse selon la revendication 7, caractérisée en ce que le stéroïde est sous une forme ophtalmique topique.

9. Une composition pharmaceutique utile pour le traitement de l'augmentation de la pression intraoculaire associée à l'utilisation d' un antiinflammatoire stéroïdien comprenant le composé selon la revendication 1 et un excipient pharmaceutiquement acceptable.

10. Une composition pharmaceutique selon la revendication 9 comprenant également un composé béta-bloquant adrénergique.

11. Une composition pharmaceutique selon la revendication 10 dans laquelle le composé béta-bloquant adrénergique est le timolol maléate.

12. Une composition pharmaceutique utile pour le traitement de la rétinopathie diabétique comprenant

le composé selon la revendication 1 et un excipient pharmaceutiquement acceptable.